# EUROPEAN PATENT APPLICATION

(11) **EP 0 916 664 A1**
(43) Date of publication of application: **19.05.1999**
(21) Application number: 97500194.2
(22) Date of filing: 13.11.1997
(51) Int. Cl.: C07D 309/30, C07D 493/04

(54) **Enynes used in the synthesis of antifungal agents**

(71) Applicant: Glaxo Wellcome, S.A., 28760 Tres Cantos (Madrid) (ES)
(72) Inventor: Calderon, Jose Maria Bueno c/o Glaxo Wellcome S.A., 28760 Tres Cantos, Madrid (ES); Fiandor Roman, Jose c/o Glaxo Wellcome S.A., 28760 Tres Cantos, Madrid (ES)
(74) Representative: Carpintero Lopez, Francisco

(57) **Abstract**

A compound of formula (II) 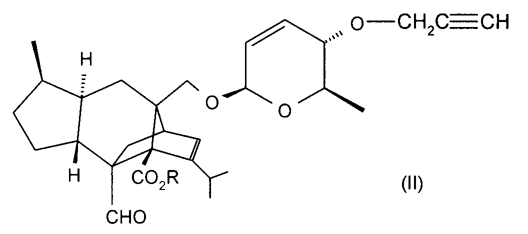 wherein R is hydrogen or a carboxyl protecting group, and its use in the preparation of the antifungal agent compound (I). 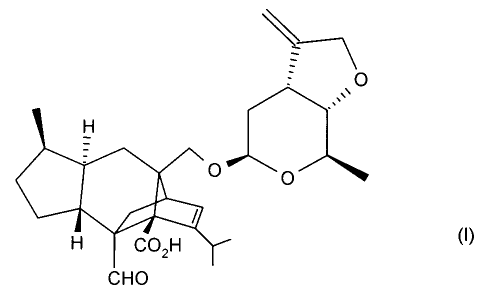 or a carboxy protected derivative thereof or a physiologically acceptable salt or metabolically labile ester thereof.

## Description

The present invention relates to novel enynes and their use in synthesis of an antifungal agent.

WO 96/14326 describes inter alia the compound of formula (I), pharmaceutically acceptable salts and metabolically labile esters thereof having a particularly desirable spectrum of antifungal activity.

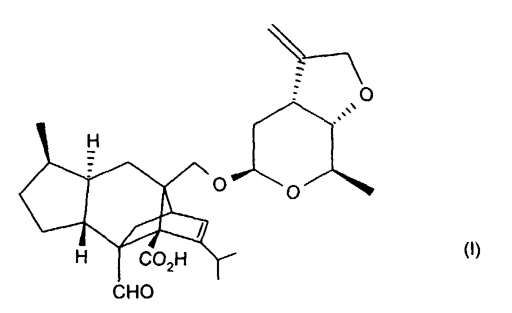

We have now found a novel group of enynes which are particularly useful for the preparation of the compound of formula (I).

Thus the present invention provides compounds of formula (II) wherein R is hydrogen or a carboxyl protecting group

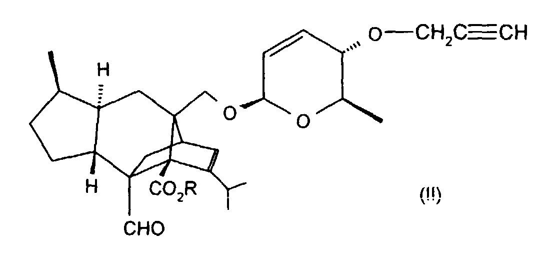

When R is a carboxyl protecting group, examples of particularly suitable protecting groups include ester groups such as substituted methyl esters. Examples of suitable substituted methyl esters include acyloxymethyl or 1-(C₁-₄alkyl)-1-acyloxymethyl wherein acyl represents C₂₋₆ alkanoyl, aroyl e.g. optional substituted benzoyl, C₂₋₆ alkanoyl substituted by an aryl group (e.g. optionally substituted phenyl), trialkylsilylethoxymethyl, allyl or propargyl. Conveniently, the carboxyl protecting group is a C₂₋₆ alkanoyloxymethyl group such as acetyloxymethyl or pivaloyloxymethyl, trimethylsilylethyloxymethyl, allyl or propargyl. Further carboxyl protecting groups useful in the synthesis of compounds of formula (II) are the substituted methyl esters such as diphenylmethyl, benzyl, p-nitrobenzyl.

Preferred compounds of formula (II) include those wherein R is hydrogen, pivaloyloxymethyl, trimethylsilylethyloxymethyl, allyl or propargyl. A particularly preferred compound is that wherein R is pivaloyloxymethyl.

In a further aspect the present invention provides an improved process for the preparation of compound (I) or a carboxyl protected thereof which comprises cyclisation of a compound of formula (II) wherein R has the meanings defined above. 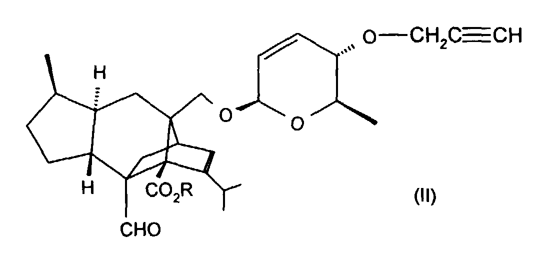 followed where desired or necessary by one or more of the following steps;
(i) removal of the carboxyl protecting group R;
(ii) isolation of the compound of formula (I) in the form of a physiologically acceptable salt thereof;
(iii) conversion of the resultant compound of formula (I) into a metabolically labile ester thereof.

In one embodiment of the process the compound of formula (I) may be prepared by a reductive cyclisation of a compound of formula (II) using a suitable palladium catalyst in the presence of a hydride donor. The reaction may be carried out in a solvent such as hydrocarbon e.g. isooctane or an aromatic hydrocarbon e.g. toluene or a halohydrocarbon such as dichloromethane or 1,2-dichloroethane. The reaction is desirably carried out at a temperature within the range 0-120° e.g. 10-110° and more conveniently at a temperature within the range 20-50°.

Suitable palladium catalysts for use in the reaction include those formed by the reaction of acetic acid with (i) a palladium salt e.g. palladium acetate with a ligand such as bis-(benzylidene)-ethylenediamine or (ii) palladium (0) complexes e.g. tris (dibenzylideneacetone) dipalladium (0) chloroform adduct in the presence of a ligand such as a bis(diphenylphosphino) derivative (e.g. 1,2-bis (diphenylphosphino)ethane or 1,1-bis-(diphenylphosphine) ferrocene) or triphenylarsine or bis-(benzylidine)ethylenediamine, or (iii) bis(1,2-bis(diphenylphosphino ethane)-palladium (0) or (iv) (1,2-bis(diphenylphosphino ethane)dichloropalladium (II) or (1,1-bis(diphenylphosphine) ferrocene) dichloropalladium (II) complex with dichloromethane (1:1). These catalysts are examples of suitable hydrido palladium catalysts that may be used in the reaction and it will be appreciated that the invention also includes to the use of other such hydrido palladium catalysts in the reductive cyclisation reaction.

Examples of suitable hydride donors include silanes such as polymethylhydresiloxane or triethylsilane.

In the cyclisation reaction it may also be convenient to carry out the process in the presence of an additional ligand or ligands such as a bis (diphenylphosphino)alkane e.g. bis 1,2-(diphenylphosphino)ethane, bis 1,1 diphenylphosphineferrocene or triphenylarsine.

For the cyclisation of a compound of formula (II) wherein R is hydrogen, the reaction may be carried out using Palladium on charcoal in the presence of a hydride donor, and optionally in the presence of acetic acid.

In a preferred embodiment of this process the cyclisation is carried using a compound of formula (II) wherein R is a propargyl group or more particularly a pivaloyloxymethyl group. In this embodiment the preferred catalysts include the product of the reaction of acetic acid with (i) a palladium salt e.g. palladium acetate with a ligand such as bis-(benzylidene)-ethylenediamine, (ii) palladium (0) complexes e.g. tris (dibenzylideneacetone) dipalladium (0) chloroform adduct in the presence of a ligand such as a bis(diphenylphosphino) derivative (e.g. 1,2 bis (diphenylphosphino)ethane or 1,1-bis(diphenylphosphineferrocene) or triphenylarsine or bis-(benzylidine)ethylenediamine, or (iii) bis (1,2-bis(diphenylphosphino) ethane) palladium (0) or (iv) (1,2-bis(diphenylphosphino) ethane)dichloropalladium (II) or (1,1-bis(diphenylphosphine)-ferrocene) dichloropalladium (II) complex with dichloromethane (1:1).

Particularly convenient solvents for use in this preferred embodiment include toluene or 1,2-dichloroethane.

In a further embodiment of the process, the cyclisation of a compound of formula (II) wherein R is a carboxyl protecting group may be carried out using a tin centered radical chain carrier in the presence of a radical initiator followed by reaction of the resultant vinyl stannane with potassium fluoride in acetic acid. A convenient source of tin centered radicals are the trialkyltin hydrides e.g. tributyltin hydride. Suitable radical initiators include azoisobutyronitrile.

The radical initiated cyclisation process to yield the vinyl stannane is conveniently carried out in an aprotic solvent and with heating e.g. 80-150°. Suitable aprotic solvents include hydrocarbons (e.g. iso-octane) or aromatic hydrocarbons e.g. toluene.

The carboxyl protecting groups may be removed by conventional procedures well known to those skilled in the art. For example pivaloyloxymethyl may be cleaved by reaction with a suitable base such as an alkali metal alkoxide e.g. sodium methoxide or potassium methoxide in a suitable solvent such as an alcohol e.g. methanol. With this process the compound of formula (I) may be either isolated directly as the free acid or as the corresponding alkali metal salt.

The trimethylsilylethyloxymethyl ester may be cleaved by reaction with fluoride ions e.g. tetrabutylammonium fluoride in a suitable aprotic solvent such as an ether e.g. tetrahydrofuran.

The allyl ester may be removed by treatment with an allyl scavenger e.g. morpholine in the presence of tetrakis (triphenylphosphine) palladium.

The propargyl ester may be reduced to the allyl ester which can then be removed by reaction with morpholine in the presence of tetrakis (triphenylphosphine) palladium. In practise if the propargyl ester is used in the reductive cyclisation reaction, the protecting group can be reduced to an allyl group in the course of the reaction.

Suitable pharmaceutically acceptable salts of the compounds of formula (I) include inorganic base salts such as alkali metal salts (for example sodium and potassium salts) and ammonium salts and organic base salts. Suitable organic base salts include amine salts such as trialkylamine (e.g. triethylamine), dialkylamine (e.g. dicyclohexylamine), optionally substituted benzylamine (e.g. phenylbenzylamine or p-bromobenzylamine), procaine, ethanolamine, diethanolamine, N-methylglucosamine and tris(hydroxymethyl)methylamine salts and amino acid salts (e.g. lysine and arginine salts).

Salts of compounds of formula (I) may be conveniently formed by treating a compound of formula (I) with an appropriate salt or base. Thus, for example, salts may conveniently be prepared by treating a compound of formula (I) with a salt or a base selected from sodium or potassium hydroxide, hydrogen carbonate, carbonate or acetate (e.g. potassium hydroxide, potassium hydrogen carbonate, sodium hydrogen carbonate or potassium acetate), ammonium acetate, calcium acetate or an organic amine e.g. L-lysine as appropriate. The salt may, for example, be prepared by adding the appropriate salt or base (if necessary as an aqueous solution) to a solution or suspension of the compound of formula (I) in a suitable solvent such as an alcohol (e.g. methanol) or dioxane at temperatures of for example 0°C to 80°C and conveniently at about room temperature

Pharmaceutically acceptable salts may also be prepared from other salts including other pharmaceutically acceptable salts of the compounds of formula (I), using conventional methods.

The compound of formula (II) may be prepared from the ditrifluromethanesulphonate ester (III) wherein R is a carboxyl protecting group, 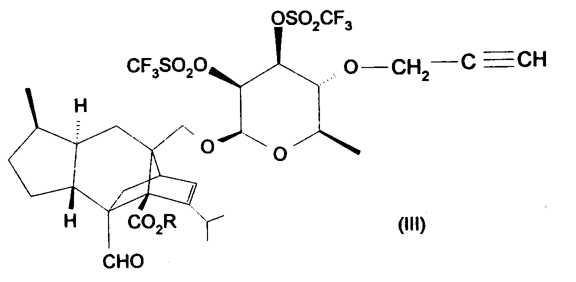 by reaction with sodium iodide in the presence of zinc, triethylamine or diethylisopropylamine in a solvent such as methylethylketone, acetone dimethoxyethane or dimethylformamide, and preferably with heating.

The ditrifluoromethanesulphonate ester (III) may be prepared from the corresponding diol (IV) wherein R is a carboxyl protecting group. 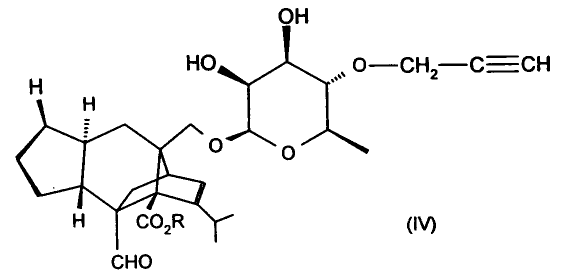 by reaction with trifluoromethanesulphonic anhydride in presence of a tertiary organic base such as pyridine and in an aprotic solvent such as dichloromethane.

The compound of formula (IV) may be prepared from the corresponding acetonide (V). 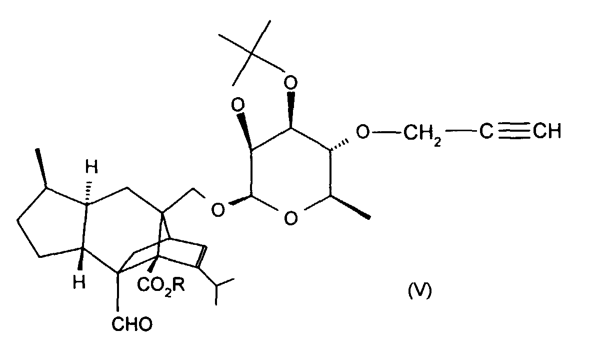 by reaction with acidic aqueous methanol, e.g. hydrochloric acid in methanol.

The propargyl ether (V) may be prepared from the corresponding alcohol (VI) 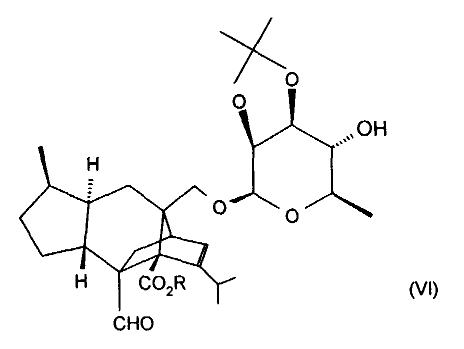 by reaction with propargyl bromide in a solvent such as dichloromethane or t-butylmethylether and in the presence of aqueous sodium hydroxide and a phase transfer catalyst such as tricaprylylmethylammonium chloride. If this reaction is carried out with an alcohol (VI) wherein R is hydrogen the resultant product is a compound of formula (V) wherein R is a propargyl group.

The compound of formula (VI) may be prepared by reaction of the triol (VII) with p-toluenesulphonic acid and 2,2-dimethoxypropane in acetone as solvent.

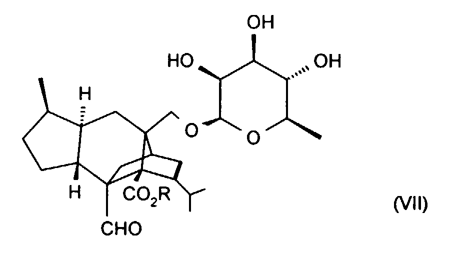

The triol (VII) wherein R is a carboxyl protecting group may be prepared from the corresponding compound (VII) wherein R is hydrogen using conventional procedures.

In the synthesis of compound (II) from triol (VII) it may be desirable to change the nature of the carboxyl protecting group R at a particular step in the synthesis and such changes are within the scope of the present invention.

Thus for the preparation of a compound of formula (II) wherein R is a pivaloyl oxymethyl group, it is desirable to introduce this protecting group at a late stage in the synthesis rather than starting with the corresponding triol (VII) wherein R is pivalolyloxymethyl. For example the synthesis may be started with the triol of formula (VII) wherein R is a diphenyl methyl group to yield the corresponding compound of formula (V) using the general synthesis described above. The diphenylmethyl ester may then be cleaved by conventional means e.g. by reaction with trifluoroacetic acid to yield the corresponding acid which can then be converted into the pivaloyloxymethyl ester by reaction with pivaloyloxymethyl chloride in the presence of a base such as diethylisopropylamine.

The compound of formula (II) wherein R is hydrogen may be prepared from the corresponding compound of formula (II) wherein R is a carboxyl protecting group using conventional means such as those described above for removing the protecting group.

The trio of formula (VII) wherein R is hydrogen may conveniently be prepared according to the fermentation process described hereinafter.

The fermentation process comprises cultivating a micro-organism capable of producing the compound of formula (VII; R=H) and thereafter isolating the compound of formula (VII; R=H) or a salt thereof e.g. a lithium salt from the culture in substantially pure form.

Microorganisms capable of producing the compound of formula (VII); R=H) will conveniently be mutant strains of Sordaria araneosa which can readily be identified by screening survivors of mutagenesis by analysing a test sample obtained from fermentation of the microorganism using standard methodology. In particular, the microorganism to be conveniently used is a mutant strain of Sordaria araneosa deposited in the permanent culture collection of the CAB International Mycological Institute, Genetic Resource Reference Collection, Bakeham Lane, Egham, Surrey TW20 9TY, England. The strain was received by the Institute on 8 December 1995 and was subsequently given the accession number IMI 369677 and confirmation of viability on 15 December 1995. The Institute is an International Depository authority recognised under the Budapest Treaty.

The microorganism IMI369677 was isolated following N-methyl-N'-nitro-N-nitrosoguanidine (MNNG) mutagenesis of ascospores of IMI362184, which is itself a mutant of NRRL3196, derived by MNNG mutagenesis. The characteristics of IMI369677 are essentially similar to those described in British Patent specification No. 1,162,027 for NRRL3196, except that IMI369677 produces Compound (VII, R=H) as a major product under the same conditions used for sordarin production by NRRL3196.

The following examples hereinafter illustrate aspects of the present invention and are not intended to limit the invention in any way

### INTERMEDIATE 1

### [1R-(1α,3aβ,4β,4aβ,7β,7aα,8aβ)]8a[6-deoxy-β-D-mannopyranosyloxy)methyl]-4-formyl-4,4a,5,6,7,7a,8,8a-octahydro-7-methyl-3-(1-methylethyl)-1,4-methano-s-indacene-3a(1H) carboxylic acid.

*Sordaria araneosa* (IMI369677) was grown on Potato Dextrose agar until mature growth occurred. Two 6mm plugs of the agar containing the growth were transferred to a 250ml Erlenmeyer flask containing 50ml of medium FS.

| Medium FS | g/L |
|---|---|
| Peptone(Oxoid L34) | 10 |
| Malt extract (Oxoid L39) | 21 |
| Glycerol (Glycerine CP) | 40 |
| Junlon 110 (Honeywell & Stein) | 1 |
| Distilled water | |

The culture was incubated at 25C for 6 days on a rotary shaker operated at 250 rpm with a 50mm diameter orbital motion. The broth was mixed 1:1 with 20% glycerol, dispensed in 1ml aliquots and stored at -80C.
A 1ml suspension prepared as described above was used to inoculate a 250ml Erlenmeyer flask containing 50ml of medium FS. The culture was incubated at 25C for 5 days on a rotary shaker operated at 250 rpm with a 50mm diameter orbital motion. 3% (v/v) portions of the developed inoculum were used to inoculate further 250 ml Erlenmeyer flasks containing medium FS (50ml) and incubated as described above for 4 days.
120 ml of the bulked shake flask developed inoculum was used to inoculate 2x7L fermenters containing 5L of medium BSM53A4. The fermentations were controlled to a temperature of 25C. The cultures were agitated at 400 rpm and aerated at 2 Lpm. After 3 days fermentation, 10 L of bulked culture was used to inoculate a 780 L fermenter containing 450 L of medium BSM53A4.

| **Medium BSM53A4** | | |
|---|---|---|
| **Ingredient** | **Supplier** | **g/L** |
| NH4H2PO4 | Fisons(Ar) | 10 |
| Glucidex32D | Roquette | 60 |
| Arkasoy | British Arkady | 20 |
| Dried CSL | Roquette | 16 |
| Meritose^ | Tunnel Refineries | 44 |
| PPG2000 | K & K Greef | 0.5 |
| Demineralised H2O Natural pH (in the range 4.0-4.5) | | |

| | | |
|---|---|---|
| ^Meritose sterilised separately as a 70% solution and added to batch post-sterilisation. | | |

The fermentation was controlled to a temperature of 25C. The broth was agitated at 350 rpm and aerated at 450 Lpm. PPG2000 antifoam was added on demand to control foaming. Whole broth extracts were assayed for the presence of title compound by reverse phase HPLC. The culture was harvested after 11 days when the extract of a broth sample indicated a titre of 1.26g/L for the title compound.

### Isolation

Fermentation broth produced as described above was adjusted to pH9 with sodium hydroxide and, after 1hr at ambient temperature, was filtered through Dicalite on a rotary vacuum filter (1% Dicalite was added to the broth as filter aid). The filtrate was adjusted to pH 4-5 with conc. sulphuric acid and the solution applied to a column of XAD-16 resin (25L). The resin bed was washed with 0.1% phosphoric acid (10 column volumes) followed by acetonitrile:water 1:9 (10 column volumes) before the product was eluted with acetonitrile:water 60:40(2 column volumes). Flow rate throughout the process was between 1-2 column volumes per hr. The eluate was concentrated to leave an aqueous residue of 5L and a water-insoluble oil/tar. The aqueous residue was discarded. The oily residue, containing the target compound, was dissolved in acetone (3.75L) and the solution filtered. A portion of this solution was evaporated to dryness, redissolved in ethyl acetate and the solution decanted leaving a brown residue. The ethyl acetate solution (1.46L) was passed down a bed of Whatman DE-52 ion-exchange cellulose (1 .2kg) prepared in water-saturated ethyl acetate. The title compound was present in the column effluent whilst impurities were retained. The bed was given a displacement wash with ethyl acetate. Fractions containing the title compound were combined to give a solution (1.64L) containing title compound) (202g).

### Isolation of title compound as a free acid

The ethyl acetate solution from the above was extracted with 5 x 1L portions of phosphate buffer (0.2M Na₂HPO₄:0.2MKH₂PO₄ 1:0.6) and the extracts combined. The combined aqueous extracts (pH 6.68) were washed with ethyl acetate (2.5L). The spent ethyl acetate solution was combined with the ethyl acetate wash for further processing (see below). The washed aqueous extract was adjusted to pH 4.1 with H₃PO₄ and extracted with ethyl acetate (1 x 1/2volume followed by 1 x 1/3volume). The combined ethyl acetate extracts were washed with water (1L) then evaporated to dryness to yield the title compound, (82g).

### INTEMEDIATE 2

### [1R-(1α,3aβ,4β,4aβ,7β,7aα,8aβ)]8a[6-deoxy-β-D-mannopyranosyloxy)methyl]-4-formyl-4,4a,5,6,7,7a,8,8a-octahydro-7-methyl-3-(1-methylethyl)-1.4-methano-s-indacene-3a(1H) carboxylic acid, diphenylmethyl ester.

### Part a

A mixture of benzophenone hydrazone, (196.25g), sodium acetate (82.03), a 1% w/v solution of iodine in dichloromethane (40ml), tricaprylyl methyl ammonium chloride (12.13g), dichloromethane (400ml) and water (200ml), was cooled to *ca* 0°, stirred rapidly and treated simultaneously with 36-40% w/v peracetic acid in acetic acid solution (210ml) and 23.5% w/v aqueous sodium hydroxide solution (520ml), at such a rate as to maintain the temperature at 0-5° and the pH at 9.5-10.5. When the addition was complete the mixture was stirred at 0-5° for a further 0.5h. The mixture was then allowed to stand for at least 30min to allow the phases to separate. The organic phase was separated and the aqueous phase was extracted with dichloromethane (250ml). The combined organic phase was washed with 5% w/v sodium thiosulphate solution (835ml), back extracting with dichloromethane (250ml). The combined organic phase was then washed with saturated brine (835ml) and back extracted with dichloromethane (250ml), to give a 0.97M solution of diphenyldiazomethane in dichloromethane.

### Part b

A solution of intermediate 1 (294.54g) in acetone (1.47l) was cooled to 15° and then treated with a solution of diphenyldiazomethane (0.707 mol) in dichloromethane (900ml) over 30-40min washing in with a little dichloromethane. After 24h the reaction mixture was treated with acetic acid (18ml) and then heated at gentle reflux until all the purple colour was discharged. The straw coloured mixture was then concentrated *in vacuo* to *ca* 735ml and acetone (294ml) added. The stirred mixture was then treated with iso-octane (2.94L), seeding after the first 0.9L. The mixture was then cooled to 5-10° and after 3h the product is collected by vacuum filtration, is washed with iso-octane / acetone (5:1) (3 x 300ml) and is dried *in vacuo* at 35° to give the title compound. 379.82g.

### INTERMEDIATE 3

### [1R-(1α, 3aβ, 4β, 4aβ, 7β, 7aα, 8aβ)] 8a-[6-Deoxy-2,3-O-isopropyliden-β-D-mannopyranosyloxy methyl]-4-formyl-4,4a,5,6,7,7a,8,8a-octahydro-7-methyl-3-(1-methylethyl)-1,4-methano-s-indacene-3a(1H)-carboxylic acid, diphenylmethylester

A stirred solution of Intermediate 2 (1.5kg) in acetone (5.25l) was treated with 2,2-dimethoxypropane (1.35l) and (±)-camphorsulphonic acid (54g) and the resulting mixture was then stirred at 15-22°. After 1.5h the mixture was concentrated to *ca*. 3.00l, ethyl acetate (7.5l) added and the mixture again concentrated to *ca*. 3.00l. Ethyl acetate (7.5l) was then added and the mixture washed with 8%w/v aqueous sodium hydrogen carbonate (3.00l) and 9%w/v aqueous brine (2.63l). The organic phase was then separated off and concentrated to *ca*. 1.88l. The viscous oil was then treated with *iso*-octane (1.50l), seeding at this point. More iso-octane (8.25l) was added at a rate of 200ml/min. once the crystallisation has appeared to have stopped. The slurry was then cooled to 0-8°C and stirred for 1.5h, before collecting by vacuum filtration. The bed was then washed with *iso*-octane/EtOAc (26:1) (3x1.5l), briefly pulled dry, and dried in vacuum oven at 35°C to give the title compound 1.273kg.

### INTERMEDIATE 4

### [1R-(1α, 3aβ, 4β, 4aβ, 7β, 7aα, 8aβ)] 8a-[4-O-(2-propynyl)-6-deoxy-2,3-O-isopropylidene-β-D-mannopyranosyloxymethyl]-4-formyl-4,4a,5,6,7,7a,8,8a-octahydro-7-methyl-3-(1-methylethyl)-1,4-methano-s-indacene-3a(1H)-carboxylic acid, diphenylmethylester.

To a rapidly stirred mixture of the Intermediate 3, (723g) and tricaprylyl methyl ammonium chloride (14.5g) in t-butylmethylether (2.6l) and 47% w/v aqueous sodium hydroxide solution (2.6l) under nitrogen was added an 80% w/v solution of propargyl bromide in toluene (275ml). The mixture was then heated at reflux, and after 225 minutes the reaction mixture was allowed to stand and the organic phase was separated. The aqueous phase was extracted with t-butylmethylether (2 x 1.5l). The combined organic phases were washed with 2M hydrochloric acid (1.5l) and then 9% brine (1.5l). The organic phase was concentrated *in vacuo* to 2.6l and then treated with iso-octane (3.5l) and concentrated *in vacuo* to 2.5l, during which time crystallisation occured. The mixture was diluted with iso-octane (3.5l), cooled to 10°C and after 1h the product is collected by vacuum filtration, washed with iso-octane (3 x 750ml), briefly pulled dry and then dried *in vacuo* at 30° to give the title compound 645.4g.

### INTERMEDIATE 5

### [1R-(1α, 3aβ, 4β, 4aβ,7β, 7aα, 8aβ)]-8a-[[[6-Deoxy-4-O-(2-propynyl)-β-D-mannopyranosyl]oxy]methyl]-4-formyl-4,4a,5,6,7,7a,8,8a-octahydro-7-methyl-3-(1-methylethyl)-1,4-methano-s-indacene-3a(1H)-carboxylic acid

Intermediate 4 (340g) was dissolved in dichloromethane (1.35L) and then cooled to 5°. Trifluoroacetic acid (675ml) was added over 15 min, maintaining a temperature below 6°. The solution was stirred for 2 hours and then a 1:1 methanol:water mixture (100ml) was added, maintaining a temperature below 7°, and stirred for 2.5 hours.

The reaction mixture was washed with water (3x675ml) and the aqueous layers were combined and back extracted with dichloromethane (400ml). The combined dichloromethane layers were washed with saturated sodium bicarbonate solution (675ml) and the aqueous layer was back extracted with dichloromethane (100ml). The combined dichloromethane layers were concentrated *in vacuo* to a dark, viscous oil.

The oil was dissolved in sodium hydroxide solution (0.25M, 2.65L) and was washed with t-butylmethylether (3x675ml). The combined t-butylmethyl ether layers were back extracted with water (2 x 250vol). The combined aqueous layers were treated with dilute hydrochloric acid (5N, 120ml) until the pH was 3-4. The mixture was extracted with ethyl acetate (3 x 500ml). The combined ethyl acetate layers were concentrated *in vacuo* to give the title compound (253g) as a dark oil.

### INTERMEDIATE 6

### [1R-(1α.,3a.β.,4.β.,4a.β.,7.β.,7a.α.,8a.β.)]-8a-[[[6-deoxy-4-O-(2-propynyl)-β-D-mannopyranosyl]oxy]methyl]-4-formyl-4,4a,5,6,7,7a,8,8a-octahydro-7-methyl-3-(1-methylethyl)-1,4-methano-s-indacene-3a(1H)-carboxylic acid, pivaloyloxymethylester.

Intermedtate 5 (5.17g 10mmol) was dissolved in methyl ethlyketone (36ml) and sodium iodide (150mg, 1mmol), chloromethylpivalate (1.44ml, 10mmol) and potassium carbonate (967mg, 7mmol) were added. The resultant suspension was heated to *ca*. 65° under nitrogen for 3.5 hours. The mixture was cooled to 40° and treated with water (36ml) and when all the solid had dissolved the aqueous phase was separated. The aqueous phase was back-extracted with ethyl acetate (2 x 25ml). The combined organic phases were washed with sodium thiosulphate solution (5% w/v, 2 x 25ml) and water (25ml). The solution was then concentrated *in vacuo* to give the title compound as a cream foam (6.175g).

### INTERMEDIATE 7

### [1R-(1α, 3aβ, 4β, 4aβ, 7β, 7aα, 8aβ)] 8a-[[[6-deoxy-4-O-(2-propynyl)-2,3-bis-O-[(trifluoromethyl)sulfonyl]-β-D-mannopyranosyl]oxy]methyl]-4-formyl-4,4a,5,6,7,7a,8,8a-octahydro-7-methyl-3-(1-methylethyl)-1,4-methano-s-indacene-3a(1H)-carboxylic acid, pivaloyloxymethyl ester.

Pyridine (219ml) was dissolved in dichloromethane (1.89l) and cooled to 5° under nitrogen. Trifluoromethanesulphonic acid anhydride (202ml) was cautiously added, over 30 min, maintaining a temperature below 10°. Once addition was complete the solution was stirred for 15 min. A solution of intermediate 6 (271g) in dichloromethane (677ml) and added over 15 min to the solution, maintaining a temperature below 10°. The resultant solution was stirred for 135 min at 5°,
then transferred to a separator and washed successively with water (810ml), 1N hydrochloric acid (810ml) and sodium bicarbonate solution (8%w/v, 810ml). The dichloromethane solution was then concentrated *in vacuo* to give the crude title compound 391g as an oil/foam.

The crude product (328g) was dissolved in methanol (656ml) and water (98ml) was added over 15 min. The resultant slurry was stirred for 2 hours and the crystalline material was then harvested by vacuum filtration, washed with 10% water in methanol (2 x 250ml) and then dried *in vacuo* for 60 hours to give the title compound (195.6g).

### INTERMEDIATE 8

### [1R-[1α, 3αβ, 4β, 4aβ, 7β, 7aα, 8aβ (3aR*,5R*,7R*,7aS*)]]-4-formyl-4,4a,5,6,7,7a,8,8a-octahydro-7-methyl-3-(1-methylethyl)-8a-[[[3-[[tris(1,1-dimethylethyl)stannyl]ethylene]-hexahydro-7-methyl-2H-furo[2,3-c]pyran-5-yl]oxy]methyl]-1,4-methano-s-indacene-3a(1H)-carboxylic acid pivaloyloxymethyl ester.

The product of Example 1 Method B (142g) was dissolved in toluene (2.0l) and tributyltin hydride (87ml) and azoisobutyronitrile (3.5g) were added. The mixture was heated at 80°C for 45min, cooled to 40°C and the solution concentrated *in vacuo*. The residue was dissolved in iso-hexane (2.0l) and was washed with acetonitrile (3x200ml). The iso-hexane solution is concentrated *in vacuo* to give the crude title compound (211g).

### EXAMPLE 1

### [1R-[1α, 3aβ, 4β, 4aβ, 7β, 7aα, 8aβ (2R*,5S*,6R*)]]-8a-[[[5,6-dihydro-6-methyl-5-(2-propynyloxy)-2H-pyran-2-yl]oxy]methyl]-4-formyl-4,4a,5,6,7,7a,8,8a-octahydro-7-methyl-3-(1-methylethyl)-1,4-methano-s-indacene-3a(1H)-carboxylic acid, pivaloyloxymethyl ester.

### METHOD A

Sodium iodide (9.6g) is added to a solution of Intermediate 7 (15.44g) and zinc dust (6.90g) in DMF (46ml) and heated to 70° for 80min. When the reaction was complete the mixture was cooled to ambient, stirred with Harborlite (5g) and filtered through Harborlite. The filter bed was washed with ethyl acetate (120ml) and the filtrates were washed with 10% w/v aqueous lithium chloride solution (3x40l) and then with water (40ml). The organic phase was then dried over magnesium sulfate prior to concentration *in vacuo* to give the title compound as an oil (10.05g)..

### METHOD B

Intermediate 7 (1.817Kg) was dissolved in methyl ethyl ketone (14.1l) and N,N-diisopropylethylamine (1.21l) and sodium iodide (0.98Kg) were added. The resultant mixture was stirred at 81°C for 30min and then cooled. Once the mixture cooled to 40°C it was divided into 2 equal portions.
One portion was transferred to a separator, diluted with toluene (8l) and washed successively with 1N HCl(aq) (2x3.75l), water (5l), potassium bicarbonate solution (8%, 2.25l). The dark solution was then concentrated *in vacuo* to a dark oil.
The second portion was transferred to a separator, diluted with toluene (8l) and washed successively with 1N HCl(aq) (3x2.3l), water (2x3l), potassium bicarbonate solution (8%, 2.25l). This was then added to the oil obtained from the first portion and concentrated in vacuo to a dark oil.
The crude title compound (1.361Kg) was used without further purification.

### EXAMPLE 2

### [1R-(1α,3aβ,4β,4aβ,7β,7aα,8aβ)]8a[[1S,7R,9R]-2,8-dioxa-9-methyl-4-methylene-cis-bicyclo[3,4,0]-non-7-yl-oxymethyl]-4-formyl-4,4a,5,6,7,7a,8,8a-octahydro-7-methyl-3-(1-methylethyl)-1.4-methano-s-indacene-3a(1H) carboxylic acid, pivaloyloxymethyl ester.

### METHOD A

A solution of Example 1 (597mg) in toluene (30ml) was treated with palladium acetate (11mg), bis(benzylidene) ethylene diamine (24mg), triethylsilane (0.85ml) and acetic acid (0.1'2ml) under nitrogen and the mixture heated to 65-75°. After 0.8h the reaction mixture was allowed to cool to ambient temperature and Harborlite™ J-2 filter aid (180mg) was added and the mixture stirred for 5-10min prior to filtration through a short bed of Harborlite™ J-2 filter aid. The filter bed was washed with toluene (3 x 5ml) and the combined filtrates were washed with saturated aqueous potassium bicarbonate solution (2 x 10ml) and then with water (2x 10ml). The resultant solution was concentrated *in vacuo* and the resultant oil was purified by flash chromatography on silica (230-400 mesh) developing and eluting with 4:1 iso-hexane - ethyl acetate. These fractions containing the desired product were combined and concentrated *in vacuo* to give the title compound as a solid (350mg)

### METHOD B

Intermediate 8 (211g) was dissolved in toluene (1.20l) and acetic acid (800ml) and potassium fluoride (211g) were added. The mixture was stirred and heated to 80°C and allowed to cool overnight for 15 h before being reheated to 80°C for 15 min and then allowed to cool to 25°C. The suspension was passed through harborlite J-2 filter aid, and the harborlite plug was washed with ethyl acetate (2x400ml). The combined filtrate and washings were washed with water (3x800ml) and sodium bicarbonate solution (8%w/v, 400ml). The resultant solution was treated with iodine in toluene solution (0.5M, 65ml) and stirred for 5min. 1,8-Diazabicyco [5.4.0]undec-2-ene (200ml) was added and the resultant mixture stirred at 22°C for 1 hour. The solution was washed with water (800ml) and then passed through silica gel (230-400 mesh, 0.3Kg) under vacuum. The silica gel was washed with ethyl acetate (2x500ml). The filtrate and washings were treated with 1,8-diazobicyclo [5.4.0]undec-7-ene (100ml) for 20min, washed with water (500ml) and passed through silica gel (230-400 mesh, 1.5vol) under vacuum. The silica gel was washed with ethyl acetate (2x500ml).

The combined filtrate and washings were concentrated *in vacuo* to give a brown oil.

The oil was purified by chromatography using a gradient elution from 10% ethyl acetate in isohexane. The fractions containing the required product were concentrated *in vacuo* to give a yellow oil which was dissolved in methanol (300ml) with warming. The solution was stirred for 90min at 5°C during which time crystallisation occured. The crystals were harvested by vacuum filtration and washed with cold (10°C) methanol (2x60ml) and dryed at 30°C for 15 hours to give the title compound 36.22g)

### EXAMPLE 2

### [1R-(1α,3aβ,4β,4aβ,7β,7aα,8aβ)]8a[[1S,7R,9R]-2,8-dioxa-9-methyl-4-methylene-cis-bicyclo[3,4,0]-non-7-yl-oxymethyl]-4-formyl-4,4a,5,6,7,7a,8,8a-octahydro-7-methyl-3-(1-methylethyl)-1.4-methano-s-indacene-3a(1H) carboxylic acid, pivaloyloxymethyl ester.

### EXAMPLE 3

### [1R-(1α, 3aβ, 4β, 4aβ, 7β, 7aα, 8aβ)] 8a-[[1S,7R,9R]-2,8-Dioxa-9-methyl-4-methylene-cis-bicyclo[3,4,0]-non-7-yl-oxymethyl]-4-formyl-4,4a,5,6,7,7a,8,8a-octahydro-7-methyl-3-(1-methylethyl) -1,4-methano-s-indacene-3a(1H)-carboxylic acid.

Example 2 (372mg) was dissolved in methanol (7.2ml) and 25% potassium methoxide in methanol (0.48ml) was added, and the mixture was stirred at 20 - 25°C for 35min. The mixture was concentrated to a white residue, which was dissolved in water (4.0ml) and ethyl acetate (4ml) and was acidified to pH 4.3 using *ca* 2M HCl. The ethyl acetate layer was collected and the aqueous phase was extracted with ethyl acetate (4ml). The combined organic phases were concentrated *in vacuo* to give the title compound as a white solid 321mg.

### EXAMPLE 4

### [1R-[1α, 3aβ, 4β, 4aβ, 7β, 7aα, 8aβ.(2R*,5S*,6R*)]]-8a-[[[5,6-dihydro-6-methyl-5-octahydro-7-methyl-3-(1-methylethyl)-1,4-methano-s-indacene-3a(1H)-carboxylic acid propargyl ester. (2-propynyloxy)-2H-pyran-2-yl]oxy]methyl]-4-formyl-4,4a,5,6,7,7a,8,8a-

To a solution of Intermediate 1 (2.5 g) in dry acetone were added at room temperature dimethoxypropane (2.5 ml) and p-toluenesulphonic acid (41 mg) and the mixture stirred at room temperature until completion of the reaction (TLC control, dichloromethane/methanol v/v 20:1). The solvent was removed under vacuum and the crude dissolved in ethyl acetate (50 ml), washed with water (3x50 ml) and brine (50 ml), then dried over Na₂SO₄. The drying agent was removed by filtration and the filtrate concentrated under vacuum. Dry tetrahydrofuran 100ml was added and then tetrabutylammonium iodide (192 mg, 10%) was added to the solution. Sodium hydride (60% dispersion in mineral oil, 520 mg, 2.5 equiv) was added portionwise at room temperature. Once the gas evolution ceased, the yellowish suspension was heated to reflux for 1 h, then it was cooled carefully to 0°C (ice-water bath) under nitrogen. Propargyl bromide (80% sol. in toluene, 1.7 ml, 3 equiv) was added dropwise via syringe and the mixture was stirred at room temperature overnight. Water (50 ml) was added carefully and the solution concentrated to half the volume under vacuum. Ethyl acetate (50 ml) was added and the mixture partioned. The organic layer was washed with water (100 ml) and brine (2x50 ml), dried over Na₂SO₄. The solvent was removed and the product was purified by filtration through a short column of silica, which was eluted with mixtures of Hexane/Ethyl acetate v/v 15:1 and 10:1). The syrup obtained after elimination of the solvent in the appropriate fraction was dissolved in tetrahydrofuran/MeOH (30 ml/15 ml) and 1N HCl (15 ml) was added dropwise over a period of 5 min. The mixture was heated to 50°C until completion (TLC control, Hexane/acetone v/v 2:1). Solid sodium carbonate was added carefully until no CO₂ evolution was observed, then it was poured onto water (50 ml) and ethyl acetate (50 ml) was added. The organic layer was washed with water (2x50 ml). The aqueous layer was extracted with ethyl acetate and the combined organic layers dried over Na₂SO₄ and concentrated to dryness and the resultant product dissolved in dry dichloromethane (30 ml).

A solution of trifluoromethane sulphonic acid anhydride (2.65 ml) in dry dichloromethane (10 ml) was added dropwise over a period of 5 min to a solution of pyridine (1.64 ml) in dry DCM (50 ml). After stirring for 30 min at 0°C the dry dichloromethane solution (30 ml) prepared above was added dropwise via dropping funnel. The mixture was stirred for 45 min at 0°C and then kept in the freezer overnight. At this point the reaction was completed. The orange solution was poured onto 1N HCl (100 ml) and the two phases partioned. The organic layer was washed with 1N HCl (100 ml), 10% NaHCO₃ (2x100 ml) and brine (100 ml), then dried over Na₂SO₄. Filtration of the drying agent and removal of the solvent gave a foam after drying under vacuum. The foam was dissolved in dry DMF (60 ml) and to this solution were added sodium iodide (4.8 g) and Zinc metal (3.3 g) and the flask immersed in an oil bath preheated to 80-90°C. After 45 min. The suspension was filtered off and the solid washed with EtOAc (100 ml). The combined organic solutions were washed with 1N NH₄Cl (2x100 ml), sodium metabisulfite (2x100 ml, 10% w/w) and brine (100 ml) then dried over Na₂SO₄. Removal of the solvent gave the crude title compound which was purified by flash chromatography (Silica gel, Hexane/ethyl acetate v/v 20:1,18:1 and 15:1) to furnish the title compound as a transparent oil.

### EXAMPLE 5

### [1R-(1α, 3aβ, 4β, 4aβ, 7β, 7aα, 8aβ)] 8a-[[1S,7R,9R]-2,8-Dioxa-9-methyl-4-methylene-cis-bicyclo[3,4,0]-non-7-yl-oxymethyl]-4-formyl-4,4a,5,6,7,7a,8,8a-octahydro-7-methyl-3-(1-methylethyl) -1,4-methano-s-indacene-3a(1H)-carboxylic acid.

To a degassed solution (Ar bubbling for 10 min) of Example 4 (52 mg) in dry toluene (5 ml). Bis(1,2-(bis(diphenylphosphino)ethane palladium (0). (2.25 mg, 2.5% mol) and polymethylhydrosiloxane(24 ml, 4 equiv.) were added at once and the mixture stirred for 5 min. To the resulting solution acetic acid (15 ml, 2.5 equiv.) was added dropwise via microsyringe

Once the addition was concluded the mixture was allowed to stir at room temperature for 24 h. Tetrakis (triphenylphosphine) palladium (1.15 mg, 1% mol) was added and the mixture stirred at room temperature until consumption of all the propargyl ester to furnish allyl ester (aprox. 3h), then Morpholine (87 ml, 10 equiv) was added

After stirring overnight The solvent was removed and the oily residue thus obtained was treated successively with 1N NaOH (5 ml) and hexane (10 ml) and the two phases stirred until dissolution of all the mass of reaction. The aqueous layer was washed with hexane (3x10 ml) and 1N HCl was added dropwise until pH =4-5, then it was extracted with ethyl acetate. The aqueous solution was extracted again with ethyl acetate and the combined organic layers concentrated to dryness to give a crude which was purified by flash chromatography (Silica gel, Hexane/Acetone v/v 15:1 and 9:1) to afford the title compound (28 mg) as a syrup

### EXAMPLE 6

### [1R-(1α, 3aβ, 4β, 4aβ, 7β, 7aα, 8aβ)] 8a-[[1S,7R,9R]-2,8-Dioxa-9-methyl-4-methylene-cis-bicyclo[3,4,0]-non-7-yl-oxymethyl]-4-formyl-4,4a,5,6,7,7a,8,8a-octahydro-7-methyl-3-(1-methylethyl) -1,4-methano-s-indacene-3a(1H)-carboxylic acid.

A solution of Example 1 and the silicon hydride (10 equiv.) in dry 1,2-dichloroethane (or dichloromethane) was degassed by bubbling Argon for 5 min, then bis (1,2-bis(diphenylphosphino)ethane palladium (0) (Aldrich, 2.5% mol) was added and the mixture stirred for a few minutes until all the catalyst is dissolved.

To the yellowish solution thus formed was added portionwise glacial acetic acid (1.1 equiv.) over a period of 1 h and the mixture stirred at room temperature until completion. TLC controls (hexane/ethyl acetate v/v 10:1, twice) showed it usually takes 3-4 hs.

The grey solution was poured onto a short column of silica, being eluted with dichloromethane and then with mixtures of hexane/ethyl acetate v/v 15:1 and 10:1 to afford the title compound in 62% yield.

## Claims

1. A compound of the general formula (II) 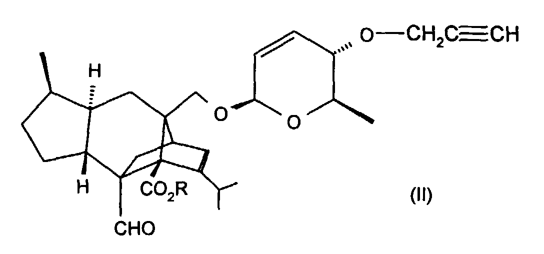 wherein R is hydrogen or a carboxyl protecting group.

2. A compound as claimed in claim 1 wherein R is a carboxyl protecting group selected from acyloxymethyl, 1-(C1-4)-1-acyloxymethyl, trialkylsilylethoxymethyl, allyl or propargyl.

3. A compound as claimed in claim 1 or claim 2 wherein R is pivaloyloxymethyl.

4. A preparation of a compound of formula (I) carboxyl protected derivative thereof. 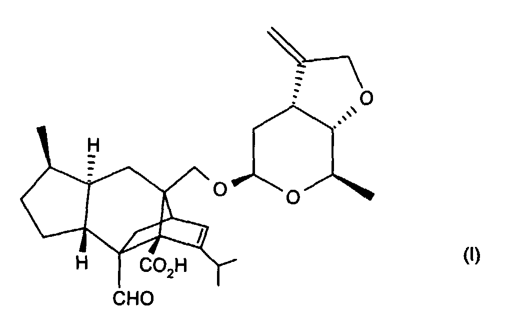 or a carboxy protected derivative thereof or a physiologically acceptable salt or metabolically labile ester thereof which comprises cyclisation of a compound of formula (II) wherein R has the meanings defined above. 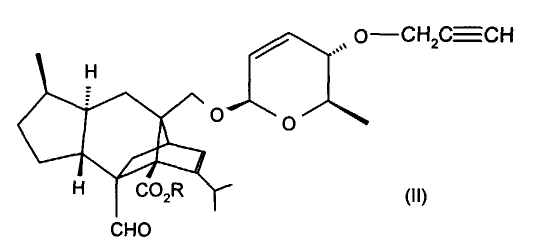 followed where desired or necessary by one or more of the following steps;
(i) removal of the carboxyl protecting group R;
(ii) isolation of the compound of formula (I) in the form of a physiologically acceptable salt thereof;
(iii) conversion of the resultant compound of formula (I) into a metabolically lable ester thereof.

5. A process as claimed in claim 4 wherein the cyclisation is effected using a suitable palladium catalyst and in the presence of a hydride donor.

6. A process as claimed in claim 5 wherein the catalyst is a hydrido palladium catalyst.

7. A process as claimed in claim 6 wherein the hydrido palladium catalyst is the product of the reaction of acetic acid with (i) a palladium salt e.g. palladium acetate with a ligand such as bis-(benzylidene)-ethylenediamine, (ii) palladium (0) complexes e.g. tris (dibenzylideneacetone) dipalladium (0) chloroform adduct in the presence of a ligand such as a bis(diphenylphosphino) derivative (e.g. 1,2 bis (diphenylphosphino)ethane or 1,1 -bis(diphenylphosphineferrocene) or triphenylarsine or bis-(benzylidine)ethylenediamine, or (iii) bis (1,2-bis(diphenylphosphino) ethane) palladium (0) or (iv) (1,2-bis(diphenylphosphino) ethane)dichloropalladium (II) or (1,1-bis(diphenylphosphine)-ferrocene) dichloropalladium (II) complex with dichloromethane (1:1).

8. A process as claimed in any of claims 4 to 7 wherein the hydride donor is a silane.

9. A process as claimed in any of claims 4 to 8 carried out in a solvent selected from toluene or 1,2-dichloroethane.

10. A process as claimed in claim 4 wherein R is a carboxcyl protecting group and the cyclisation is carried out using a tin centered radical in the presence of a radical initiator:
